# EUROPÄISCHE PATENTANMELDUNG

(11) **EP 1 048 663 A1**
(43) Veröffentlichungstag der Anmeldung: **02.11.2000**
(21) Anmeldenummer: 00104475.9
(22) Anmeldetag: 08.03.2000
(51) Int. Cl.: C07D 307/62

(54) **Verfahren zur Herstellung von L-Ascorbinsäure**

(30) Priorität: 28.04.1999 DE 19919203
(71) Anmelder: BASF AKTIENGESELLSCHAFT, 67056 Ludwigshafen (DE)
(72) Erfinder: Böttcher, Andreas, Dr., 69226 Nussloch (DE); Gurski, Hans, 67227 Frankenthal (DE); Kuntze, Thomas, Dr., 67459 Böhl-Iggelheim (DE); Kjaergaard, Karsten, 8500 Grenaa (DK)

(57) **Zusammenfassung**

Die Erfindung betrifft ein Verfahren zur Herstellung von L-Ascorbinsäure, dadurch gekennzeichnet, daß man eine Schmelze von 2-Keto-L-gulonsäure-C₃-C₁₀-alkylester unter sauren Bedingungen lactonisiert.

## Beschreibung

Die Erfindung betrifft ein Verfahren zur Herstellung von L-Ascorbinsäure, bei dem man eine Schmelze von 2-Keto-L-gulonsäure-C₃-C₁₀-alkylester unter sauren Bedingungen lactonisiert.

Zur Herstellung von L-Ascorbinsäure sind in der Vergangenheit eine Vielzahl von Verfahrensvarianten veröffentlicht worden. Eine Übersicht findet sich u.a. in Crawford et al., Adv. Carbohydrate Chem. 37, 79 (1980) sowie in Ullmann's Encyclopedia of Industrial Chemistry, Vol. A27, 551-557 (1996).

Bekannt sind eine Reihe von Verfahren zur Herstellung von Ascorbinsäure durch Umsetzung von 2-Keto-L-gulonsäure mit einer Säure.

So ist in der US 2,185,383 die Umsetzung von 2-Keto-L-gulonsäure mit konzentrierter Salzsäure und Essigsäure als Lösungsmittel beschrieben.

JP-OS 58-177986 beschreibt ein Verfahren, das die Zugabe von Ethanol und Aceton zu dem Natrium-Salz der 2-Keto-L-gulonsäure, die Neutralisation mit Salzsäure, die Abtrennung des ausgefällten Natriumchlorids durch Filtration und anschließend das Halten der Reaktionsmischung bei Temperaturen im Bereich von 25°C bis 75°C umfaßt, wodurch L-Ascorbinsäure erhalten wird.

In der JP-AS 48-15931 wird die Umsetzung von 2-Keto-L-gulonsäure mit einer Mineralsäure in einem inerten Lösungsmittel in Gegenwart einer oberflächenaktiven Substanz beschrieben.

WO 87/00839 beansprucht ein Verfahren, bei dem eine Aufschlämmung von 2-Keto-L-gulonsäure in einem inerten organischen Lösungsmittel in Gegenwart eines grenzflächenaktiven Mittel unter Säurekatalyse zu L-Ascorbinsäure umgesetzt wird.

DE-A-195 47 073 beschreibt ein Verfahren zur Herstellung von L-Ascorbinsäure durch Umsetzung von 2-Keto-L-gulonsäure mit wäßriger Mineralsäure in einem Lösungsmittelgemisch, enthaltend ein inertes organisches Lösungsmittel, ein aliphatisches Keton sowie ein Säurechlorid.

WO 99/07691 beschreibt die Umsetzung von 2-Keto-L-gulonsäure mit konzentrierter Salzsäure bei Temperaturen zwischen 40 und 80°C.

EP-A-0 671 405 offenbart ein Verfahren zur Herstellung von 2-Keto-L-gulonsäure-methyl- oder -ethylester durch Veresterung von 2-Keto-L-gulonsäure mit Methanol bzw. Ethanol in Gegenwart eines sauren Ionenaustauschers. Ferner ist in dieser Anmeldeschrift zu lesen, daß die o.g. Ester einer alkalischen Umlagerung (Lactonisierung) zur Ascorbinsäure oder zu einem Salz davon unterworfen werden können.

US 5,391,770 beschreibt die Veresterung von 2-Keto-L-gulonsäure mit anschließender basenkatalysierter Lactonisierung der gebildeten Ester zu Salzen der L-Ascorbinsäure und Freisetzung der Ascorbinsäure durch Zugabe einer starken Säure.

In der japanischen Auslegeschrift 22 113/75 wird die Veresterung von 2-Keto-L-gulonsäure mit Butanol und die anschließende säurekatalysierte Lactonisierung in Benzol als Lösungsmittel beschrieben.

Die oben genannten Ausführungsformen der sauer katalysierten, einstufigen Verfahrensvariante zeigen gravierende Schwächen. So ist in der Regel die Verwendung eines inerten Lösungsmittels unumgänglich, um die Folgereaktionen der Ascorbinsäure mit wäßriger Salzsäure zu unterdrücken. Gleichzeitig handelt man sich damit aber auch das Problem ein, daß die 2-Keto-L-gulonsäure zu Beginn und im Verlauf der Reaktion stets ungelöst in Form einer Suspension vorliegt und eine Reaktion nur an der Kristalloberfläche erfolgt. Der Zusatz von grenzflächenaktiven Substanzen ändert am Reaktionsverlauf nur wenig. Vielmehr kann dieser Hilfsstoff nur mühsam vom Rohprodukt abgetrennt werden und bedeutet zusätzliche Aufreinigungsschritte, um die gewünschte Reinheit der L-Ascorbinsäure zu erzielen. Nachteilig sind weiterhin lange Reaktionszeiten und demzufolge große Apparatevolumina.

Es bestand daher die Aufgabe, ein Verfahren zur Herstellung von L-Ascorbinsäure bereitzustellen, welches die oben genannten Nachteile nicht aufweist.

Diese Aufgabe wurde durch ein Verfahren zur Herstellung von L-Ascorbinsäure gelöst, das dadurch gekennzeichnet ist, daß man eine Schmelze von 2-Keto-L-gulonsäure-C₃-C₁₀-alkylester unter sauren Bedingungen lactonisiert.

Ferner ist das erfindungsgemäße Verfahren in einer bevorzugten Ausführungsform dadurch gekennzeichnet, daß man
a) 2-Keto-L-gulonsäure oder 2,3:4,6-Di-O-isopropyliden-2-keto-L-gulonsäure in Gegenwart eines sauren Katalysators mit einem C₃-C₁₀-Alkohol verestert,
b) den überschüssigen C₃-C₁₀-Alkohol zusammen mit dem gebildeten Reaktionswasser abdestilliert und
c) anschließend den gebildeten 2-Keto-L-gulonsäure-C₃-C₁₀-alkylester in Form einer wasserfreien Schmelze unter sauren Bedingungen lactonisiert.

Im Rahmen des erfindungsgemäßen Verfahrens wird zunächst 2-Keto-L-gulonsäure oder 2,3:4,6-Di-O-isopropyliden-2-keto-L-gulonsäure in einem einstufigen Veresterungsschritt in Gegenwart eines sauren Katalysators zum Alkylester umgesetzt. Die Veresterung erfolgt in einem Temperaturbereich von -10 bis 160°C, vorzugsweise von 20 bis 100°C, besonders bevorzugt in einem Temperaturbereich von 40 bis 90°C.

Zur Veresterung eignen sich vorteilhafterweise höhere Alkylester aus gesättigten, verzweigten oder unverzweigten Alkylalkoholen mit einer Kohlenstoffanzahl größer gleich 3, bevorzugt mit einem Alkylrest von 3 bis 10 Kohlenstoffatomen, wie z.B. n-Propanol, Isopropanol, 1-Butanol, 2-Butanol, 2-Methyl-1-propanol, 2-Methyl-2-propanol, 1-Pentanol, 2-Pentanol, 3-Pentanol, 1-Hexanol, 2-Hexanol, 1-Heptanol, 2-Heptanol, 1-Octanol, 2-Octanol, 3-Octanol, 1-Nonanol, 2-Nonanol, 1-Decanol, 2-Decanol, 4-Decanol.

Vorzugsweise werden solche Alkohole für die Veresterung eingesetzt, in denen L-Ascorbinsäure schwer löslich ist. Besonders bevorzugt sind C₄-C₈-Alkohole, ausgewählt aus der Gruppe, bestehend aus n-Propanol, Isopropanol, 1-Butanol, 2-Methyl-1-propanol, 2-Methyl-2-propanol, 1-Pentanol, 1-Hexanol und 1-Octanol, ganz besonders sind 1-Butanol und 1-Pentanol geeignet.

Der Alkohol wird dabei in einem 2- bis 10-fachen, bevorzugt 3- bis 6-fachen molaren Überschuß, bezogen auf die eingesetzte 2-Keto-L-gulonsäure bzw. 2,3:4,6-Di-O-isopropyliden-2-keto-L-gulonsäure eingesetzt.

Für die Synthese wird bevorzugt 2-Keto-L-gulonsäure als Ausgangsmaterial eingesetzt. Die Säure kann dabei sowohl in kristalliner Form, beispielsweise als getrocknetes oder schleuderfeuchtes Monohydrat oder als wasserfreie Verbindung als auch als wäßrige Lösung, beispielsweise als aufkonzentrierte Fermentationslösung eingesetzt werden.

Das Monohydrat der 2-Keto-L-gulonsäure fällt in der Regel bei der Kristallisation aus Wasser oder wasserhaltigen, organischen Lösungsmitteln an. Durch Abschleudern der Kristallmaische ist feuchtes Monohydrat zugänglich. Dieses kann als schleuderfeuchtes Produkt direkt in der nachfolgenden Veresterungsreaktion eingesetzt oder unter milden Bedingungen getrocknet werden.

Es ist auch möglich, eine aufkonzentrierte wäßrige Lösung der 2-Keto-L-gulonsäure direkt in die Veresterungsreaktion einzusetzen. Das überschüssige Lösungsmittel wird vor oder während der Veresterungsreaktion z.B. durch Extraktion und Phasentrennung oder Azeotropdestillation entfernt. Insbesondere eignet sich diese Vorgehensweise für eine Ketogulonsäure-Lösung aus einem fermentativen Herstellprozeß. Nach Abtrennung der Biomasse durch an sich bekannte Standard-Verfahren kann die meist gefärbte Fermentationslösung, vorzugsweise nach flüssig-flüssig Extraktion, ohne weitere Reinigung direkt eingesetzt werden. Das überschüssige Lösungsmittel wird danach, wie oben beschrieben, vor oder während der Veresterungsreaktion z.B. durch Phasentrennung oder Azeotropdestillation entfernt.

Wasserfreie 2-Keto-L-gulonsäure erhält man u.a. aus dem kristallinen, ggf. schleuderfeuchten Monohydrat unter verschärften Trocknungsbedingungen.

Vorteilhafterweise kann man bei dem erfindungsgemäßen Verfahren auf die Trocknung bzw. Entwässerung des Monohydrates der 2-Keto-L-gulonsäure verzichten, da bei der nachfolgenden, erfindungsgemäßen Aktivierungsreaktion ohnehin eine azeotrope Entwässerung durchgeführt wird.

Durch Zusatz einer 0,005 bis 0,1 molaren, vorzugsweise einer 0,005 bis 0,05 molaren Menge eines sauren Katalysators, in freier oder polymer gebundener Form (als stark saurer Kationenaustauscher) oder ihres Esters wird die Veresterungsreaktion katalysiert. Unter der Bezeichnung 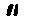 saurer Kationenaustauscher" sind kommerziell erhältliche Harze zu verstehen, wie z.B. Lewatit® S 100 und SP 112 (Bayer) oder Amberlite® 18 und IRA 120 oder Amberlyst® 15 oder Duolite® C 20, C 26 und C 264 (Rohm & Maas) oder Dowex® Ionentauscher.

Als weitere Katalysatoren sind auch Säuren bzw. deren Derivate geeignet. Dazu zählen beispielsweise Phosphorsäure, Phosphorsäure-monobutylester, Phosphorsäure-dibutylester, Phosphorsäure-monopentylester, Phosphorsäure-dipentylester, Schwefelsäure, Schwefelsäure-monobutylester, Schwefelsäure-monopentylester, Chlorwasserstoff, p-Tolsulfonsäure, Methansulfonsäure, Chlorsulfonsäure, Trifluoressigsäure und andere starke, wasserfreie Säuren.

Auch 2-Keto-L-gulonsäure, 2,3:4,6-Di-O-isopropyliden-2-keto-L-gulonsäure oder L-Ascorbinsäure können als saure Veresterungskatalysatoren eingesetzt werden.

Bevorzugt verwendet man allerdings Schwefelsäure, Methansulfonsäure oder Monoalkylsulfate der verwendeten C₃-C₁₀-Alkohole, besonders bevorzugt sei Schwefelsäure genannt. Die Monoalkylsulfate spalten bei Temperaturen oberhalb von 70°C Schwefelsäure ab [Popelier, Bull. Soc. Chim. Belg. 35, 265 (1926)], die katalytisch wirkt. Daher ist bei Einsatz dieser Katalysatoren eine Veresterung meist erst bei höheren Temperaturen möglich.

Um einen möglichst vollständigen Umsatz bei der Veresterung zu erzielen, ist es von Vorteil, das Reaktionswasser möglichst vollständig zu entfernen. Vorteilhafterweise wird im vorliegenden Verfahren das Reaktionswasser mit überschüssigem Alkohol abdestilliert. Dies erfolgt in einem Druckbereich von 20 mbar bis Normaldruck, vorzugsweise in einem Bereich von 100 bis 800 mbar. Der Alkohol dient dabei als Schleppmittel für das gebildete Reaktionswasser. Alkohole mit weniger als 3 Kohlenstoffatomen sind dafür nicht so gut geeignet. Das Destillat wird nach der Entfernung des Wassers durch Phasentrennung, destillative Trocknung oder Trocknung durch wasserentziehende Mittel, wie z.B. Molekularsiebe, für weitere Veresterungsreaktionen verwendet.

Als Lösungsmittel zur Wasserauskreisung verwendet man vorteilhafterweise den Veresterungsalkohol oder eine Mischung aus diesem Alkohol und einem weiteren mit Wasser nicht mischbaren Lösungsmittel. Die erfindungsgemäß zu verwendenden Alkohole haben nur eine begrenzte Kapazität zur Ausschleusung des Reaktionswassers. Insbesondere bei hohen Katalysatorsäure-Konzentrationen oder kurzen Veresterungszeiten ist es von Vorteil, wenn bereits bei der Veresterungsreaktion ein zweites Lösungsmittel als Wasserschlepper zugegeben wird. Dieses Lösungsmittel sollte ein niedrig siedendes Azeotrop mit Wasser bilden und ggf. mit dem Alkohol nur begrenzt mischbar sein, um so den Alkohol während der Veresterungsreaktion nach Phasentrennung rückführen zu können. Von diesem zweiten Lösungsmittel werden nur geringe Mengen, vorteilhafterweise 10 bis 50 mol-% bezogen auf 2-Keto-L-gulonsäure, benötigt.

Vorteilhafterweise werden dafür unpolare halogenierte Kohlenwasserstoffe, wie z.B. Tetrachlorkohlenstoff, Chloroform, Dichlorethan, 1,2-Trichlorethylen, Perchlorethylen oder aromatische Kohlenwasserstoffe, wie Toluol, Xylol eingesetzt. Des weiteren kann auch Propylencarbonat verwendet werden. Als bevorzugtes Lösungsmittel sei Perchlorethylen genannt.

Der Umsatzgrad der 2-Keto-L-gulonsäure bei der Veresterungsreaktion liegt im erfindungsgemäßen Verfahren deutlich über 90 %, vorzugsweise in einem Bereich von 95 bis 99 %.

Im Verlauf der Veresterungsreaktion geht die 2-Keto-L-gulonsäure in Lösung, womit man einen guten optischen Indikator für den Fortschritt der Reaktion hat. In Abhängigkeit von der Menge der eingesetzten Katalysatorsäure erfolgt dies in einem Zeitraum von wenigen Minuten bis mehreren Stunden. Höhere Temperaturen, z.B. im Bereich von 30 bis 150°C begünstigen den Umsatz bei der Veresterungsreaktion. Gegen Ende der Reaktion, wenn ein Großteil des überschüssigen Alkohols abdestilliert worden ist, wird das Reaktionsgemisch zähflüssiger. Vorteilhafterweise kann die Veresterungsreaktion als abgeschlossen betrachtet werden, wenn eine lösungsmittel- und wasserfreie Schmelze des entsprechenden Keto-L-gulonsäure-alkylesters entstanden ist. Die Viskosität dieser Schmelze ist von den Substanzeigenschaften des jeweiligen 2-Keto-L-gulonsäureesters und der Temperatur abhängig.

Das Reaktionsgemisch erstarrt beim Abkühlen in einem Temperaturbereich von 20 bis 40°C. Beim Erwärmen bildet sich die Schmelze reversibel und ohne Zersetzung zurück. Ein erneuter Zusatz von trockenem Alkohol zur Vervollständigung der Veresterungsreaktion, wie beispielsweise in WO 99-3853 beschrieben, ist unter den oben genannten Bedingungen nicht erforderlich, weil a) die Veresterungsreaktion fast vollständig erfolgt und b) ein 100%iger Umsatz gemäß der Lehre der vorliegenden Erfindung nicht zwingend erforderlich ist. Bei Verwendung des Monohydrates der Keto-L-gulonsäure ist keine höhere Alkoholmenge zu Beginn der Veresterung erforderlich. Die Veresterungsgeschwindigkeit wird durch den Restwassergehalt des zurückgeführten Alkohols bestimmt.

Anstelle von 2-Keto-L-gulonsäure kann auch 2,3:4,6-Di-O-isopropyliden-2-keto-L-gulonsäure unter den oben genannten Bedingungen in gleicher Weise verestert werden. Dabei erfolgt zusätzlich eine Abspaltung der Aceton-Schutzgruppen. Für die Abspaltung werden zwei Moläquivalente Wasser benötigt, während gleichzeitig ein Moläquivalent Wasser bei der Veresterungsreaktion gebildet wird. Dies bedeutet, daß anstelle von wasserentziehenden Mitteln und physikalischen Trocknungsverfahren auch chemische Reaktionen zur Entfernung des Reaktionswassers eingesetzt werden können. In einfachster Weise setzt man daher das Monohydrat der 2,3:4,6-Di-O-isopropyliden-2-keto-L-gulonsäure ein. Die Reaktion erfolgt ebenfalls im oben beschriebenen Druck- und Temperaturbereich.

Das gebildete Aceton wird als Leichtsieder während der Veresterungsreaktion zu Beginn bzw. zusammen mit überschüssigem, wasserhaltigem Lösungsmittel abdestilliert und kann nach Isolierung und Reingewinnung zurückgeführt werden.

Die Veresterungsreaktion von 2-Keto-L-gulonsäure bzw. 2,3:4,6-Di-O-isopropyliden-2-keto-L-gulonsäure kann diskontinuierlich oder kontinuierlich betrieben werden. Bei der kontinuierlichen Veresterung wird die Azeotropdestillation beispielsweise in einer Rührkesselkaskade, einem Dünnfilmverdampfer oder in ähnlich arbeitenden Apparaten durchgeführt. Auch unter diesen Bedingungen entsteht am Ende der Veresterung eine schmelzflüssige Form des entsprechenden 2-Keto-L-gulonsäure-alkylesters. Der Vorteil dieser Arbeitsweise besteht im Vergleich zur diskontinuierlichen Veresterung darin, daß die Reaktionszeit bei gleichem Umsatz und gleicher Reinheit deutlich unter einer Stunde liegt.

In der bevorzugten Ausführungsform des erfindungsgemäßen Verfahrens erhält man nach den Verfahrensschritten a) und b) eine Schmelze bzw. schmelzflüssige Form des Esters, die gut fließfähig ist und leicht in Rohrleitungen transportiert werden kann. Diese Schmelze kann ohne Isolierung bzw. ohne weitere Reinigung entweder unter 100°C bei Normaldruck ggf. in einem inerten Lösungsmittel, wie z.B. einem halogenierten Kohlenwasserstoff, in Gegenwart eines sauren Katalysators, wie z.B. Salzsäure, oder oberhalb von 90°C bei erhöhtem Druck in Gegenwart geringer Mengen von Wasser ohne ein weiteres organisches Lösungsmittel, ggf. in Gegenwart eines sauren Katalysators, wie z.B. Chlorwasserstoff, unter Freisetzung des zur Aktivierung verwendeten Alkohols zu L-Ascorbinsäure hoher Reinheit umgelagert werden.

Im Anschluß an die Veresterungsreaktion der 2-Keto-L-gulonsäure bzw. 2,3:4,6-Di-O-isopropyliden-2-keto-L-gulonsäure erfolgt im Verfahrensschritt c) die Umlagerung (Lactonisierung) des gebildeten Esters - in Form seiner Schmelze - zu L-Ascorbinsäure in Gegenwart eines sauren Katalysators. Bevorzugt handelt es sich dabei um eine wasserfreie Schmelze.

Gegenstand der vorliegenden Erfindung sind dabei zwei verschiedene Verfahrensweisen für die Art dieser Umlagerung. Die Umlagerung kann bei niedrigeren Temperaturen und längeren Reaktionszeiten in Gegenwart eines zusätzlichen inerten Lösungsmittels oder direkt bei höheren Temperaturen ohne Zuhilfenahme von inerten Lösungsmitteln, aber ggf. in Gegenwart von Wasser erfolgen.

Es ist bekannt, daß die Ascorbinsäure beispielsweise bei der Umlagerung mit wäßriger Salzsäure zu unerwünschten, dunkel gefärbten Nebenprodukten reagiert [Crawford et al., Adv. Carbohydrate Chem. 37, 79-155 (1980)], wenn sie nicht unmittelbar nach ihrer Bildung aus dem Reaktionsgemisch entfernt wird. Dies gelingt in der Regel bei der klassischen Lösungsmittelfahrweise durch Zusatz eines inerten Lösungsmittels, in dem die Ascorbinsäure un- oder schwer löslich ist. Unter Berücksichtigung dieser Tatsache wird verständlich, daß bei den literaturbekannten Verfahren häufig im technischen Maßstab Schwierigkeiten auftreten, weil die ausfallende Ascorbinsäure nur zu schwer rührfähigen und nicht leicht zu handhabenden Mischungen bzw. wachsähnlichen Massen führt. Insbesondere werden bei dieser Verfahrensweise beim Kristallisationsvorgang, der meist über einen amorphen Feststoff verläuft, geringe Mengen unerwünschter Katalysatorsäure eingeschlossen. Diese restlichen Mengen nicht abgetrennter Katalysatorsäure führen auch in Spuren im Endprodukt zu schlechteren Qualitäten und erfordern daher meist einen unwirtschaftlich hohen Reinigungsaufwand.

Bei Einsatz eines Esters der 2-Keto-L-gulonsäure werden bei der sauer katalysierten Umlagerung im Laufe der Reaktion äquivalente Mengen Alkohol aus dem Ester wieder freigesetzt. In dem Alkohol sind einerseits das Reaktionsprodukt, die L-Ascorbinsäure, erfindungsgemäß schwer löslich und andererseits die Katalysatorsäure sowie geringe Mengen an Nebenprodukten dagegen leichter löslich. Wird die Umlagerung des Esters von Beginn an in einem inerten Lösungsmittel durchgeführt, dann läßt sich der Reinigungeeffekt des inerten Lösungsmittels durch den freigesetzten Alkohol signifikant verstärken.

Hierzu eignen sich vorteilhafterweise die gleichen erfindungsgemäßen höheren Alkohole, wie sie zur Veresterung der Keto-gulonsäure eingesetzt werden. Dies sind insbesondere Alkohole mit einer Kohlenstoffanzahl größer gleich 3, wie z.B. n-Propanol, Isopropanol, n-Butanol, iso-Butanol, 1-Pentanol, 2-Pentanol, 3-Pentanol, 1-Hexanol, 2-Hexanol, 1-Heptanol, 2-Heptanol, 1-Octanol, 2-Octanol, 3-Octanol, 1-Nonanol, 2-Nonanol, 1-Decanol, 2-Decanol, 4-Decanol usw.

Auch die unpolaren, inerten Lösungsmittel, die sich vorzugsweise für die Umlagerung in Lösung eignen, wurden oben bereits beschrieben. Vorteilhafterweise werden unpolare halogenierte Kohlenwasserstoffe, wie z.B. Tetrachlorkohlenstoff, Chloroform, Dichlorethan, 1,2-Trichlorethylen, Perchlorethylen oder aromatische Kohlenwasserstoffe, wie Toluol, Chlorbenzol oder Xylol eingesetzt. Besonders bevorzugt ist Perchlorethylen. Mit gutem Erfolg können auch cyclische Carbonate, wie z.B. Propylencarbonat, eingesetzt werden.

Die Umlagerung erfolgt nach Verfahrensvariante a) in Lösung in einem Temperaturbereich von 40 bis 100°C, vorzugsweise zwischen 50 und 95°C. Dabei muß die Reaktionszeit der entsprechenden Umlagerungstemperatur angepaßt werden. Die Reaktionszeiten liegen im o.g. Temperaturbereich bei Normaldruck vorzugsweise zwischen 0,25 und 20 Stunden. Unter diesen Bedingungen werden hohe Ausbeuten und hohe Reinheiten der L-Ascorbinsäure erreicht.

Für den Erfolg der Umlagerungsreaktion ist die eingesetzte Katalysatorsäure von entscheidender Bedeutung. Mineralsäuren, wie z.B. Phosphorsäure oder Schwefelsäure sind geeignet. Vorteilhafterweise setzt man Chlorwasserstoff, entweder als konzentrierte wäßrige Salzsäure oder als Gas, das direkt in das Umlagerungsgemisch eingeleitet wird, ein. Für die Umlagerung wird Wasser benötigt. Daher muß bei Einsatz von Chlorwasserstoff die erforderliche Menge Wasser zugesetzt werden. Es ist von Vorteil, das Reaktionswasser nicht über die Veresterungsstufe in die Umlagerungsreaktion einzubringen. Eine zu hohe Säurekonzentration bedingt in Gegenwart von Wasser geringere Ausbeuten an Wertprodukt und schlechtere Reinheiten. Eine Konzentration von 1 bis 5 % bezogen auf Chlorwasserstoffgas in der oben angegebenen Mischung aus höherem Alkohol und dem inerten Lösungsmittel wird bevorzugt eingesetzt.

Die Katalysatorsäure kann im Umlagerungsreaktor mit dem inerten Lösungsmittel vorgelegt oder zu dem Umlagerungsgemisch zugegeben werden.

Für längere Umlagerungszeiten bei erfindungsgemäß niedrigeren Temperaturen läßt man in der Regel bevorzugt die schmelzflüssige Form des 2-Keto-L-gulonsäure-alkylesters in das vorgelegte inerte Lösungsmittel zusammen mit der Katalysatorsäure laufen oder man arbeitet invers. In allen Fällen ist eine ausreichende Durchmischung der Säure mit dem Reaktionsgemisch für den Erfolg der Umlagerung wichtig. Die gebildetet L-Ascorbinsäure fällt gegen Ende der Umlagerung in kristalliner Form aus und kann durch übliche Verfahren, wie z.B. Absaugen, Abschleudern, Abpressen oder Extraktion isoliert werden. Nach dem Waschen mit dem Alkohol, der auch für die Veresterung eingesetzt wurde, und Trocknen erhält man das Vitamin C als Rohprodukt in hohen Reinheiten (98,5 bis 99,8 %) und Ausbeuten (87 bis 93 %).

Diese Art der Reaktionsführung hat einen, für den Fachmann überraschenden Nebeneffekt, der sich auf die Reinheit und Ausbeute des Reaktionsproduktes signifikant auswirkt. Bei dieser Umlagerung werden vorteilhafterweise zusätzlich in situ gezielt geringe Mengen von aktiviertem Kohlenstoff in fein verteilter Form erzeugt, die Spuren unerwünschter Nebenprodukte adsorbieren und mechanisch leicht abgetrennt werden können. Damit wird eine aufwendige Reinigung der entstandenen L-Ascorbinsäure überflüssig. Der Anteil an aktiviertem Kohlenstoff liegt im Bereich von 0,1 bis maximal 0,8 %. Diese Mengen reichen aus, um beispielsweise gefärbte Verunreinigungen, die u.a. aus fermentativ hergestellter 2-Keto-L-gulonsäure stammen können, wirksam zu adsorbieren. Der gebildete Kohlenstoff kann im Zuge der Isolierung der Ascorbinsäure bequem abgetrennt werden.

Die Ausbeute und Reinheit an Vitamin C wird signifikant durch die Zusammensetzung des Lösungsmittelgemisches im Verlauf der Umlagerung beeinflußt. Eine zu hohe Wasserkonzentration reduziert die Ausbeute. Der Wassergehalt in dieser Mischung sollte daher im Bereich von 1 bis 10 %, vorzugsweise im Bereich von 2 bis 7 % liegen. Gegen Ende der Umlagerung enthält das Umlagerungsgemisch vorteilhafterweise 50 bis 90 Gewichtsprozent des inerten Lösungsmittels und 50 bis 10 Gewichtsprozent des gebildeten Alkohols. In dieser Mischung sind L-Ascorbinsäure schwer und alle anderen Edukte bzw. Nebenprodukte und die Katalysatorsäure leicht löslich.

Die 2-Keto-gulonsäure-alkylester Schmelze kann nach oben genannter Verfahrensvariante b) auch ohne Einsatz eines inerten Lösungsmittels umgelagert werden. Dabei wird der Veresterungsalkohol im Verlauf der Reaktion freigesetzt und bewirkt die Bildung einer feinverteilten, pumpfähigen Kristallsuspension der L-Ascorbinsäure. Diese Art der Umlagerung in Gegenwart der bevorzugten Katalysatorsäure Chlorwasserstoff bzw. Salzsäure erfolgt in wesentlich kürzerer Zeit, als bei Verfahrensvariante a) bereits beschrieben. Erfindungsgemäß wird dabei wird auf den Zusatz des inerten, organischen Lösungsmittels verzichtet. Die Anwesenheit von Wasser ist aber erfindungsgemäß vorteilhaft.

Erfindungsgemäß wird hierbei die Schmelze oder schmelzflüssige Form des 2-Keto-L-gulonsäure-alkylesters, bei Temperaturen zwischen 20 und 100°C mit der Katalysatorsäure zügig vermischt und anschließend bei einer Temperatur zwischen 95 und 160°C, vorzugsweise zwischen 100 und 140°C kurzzeitig umgelagert. Bei dieser Umlagerungsreaktion wird vorteilhafterweise bei einem Druck von 0,5 bis 10 bar gearbeitet. Die Verweilzeit im Reaktor, der beispielsweise als Rohrreaktor konzipiert werden kann, liegt vorzugsweise im Bereich von wenigen Sekunden bis hin zu 1 Stunde. Bei dieser Art der Umlagerung, die erfindungsgemäß bevorzugt auch kontinuierlich betrieben werden kann, wird der Alkohol abgespalten und anschließend bei vermindertem Druck so verdampft, daß eine Gleichgewichtseinstellung zwischen Dampf und Flüssigkeit und eine Folgereaktion der L-Ascorbinsäure mit der Katalysatorsäure möglichst vollständig vermieden wird. Durch Entspannung im Vakuum wird die Katalysatorsäure mit einer hohen Verdampfungsgeschwindigkeit entfernt und gleichzeitig L-Ascorbinsäure in feinverteilter Form im Alkohol ausgefällt. Der freigesetzte Alkohol kann nach bekannten Verfahren zurückgewonnen und für die Estersynthese erneut verwendet werden. Die als Rückstand aus dieser Umlagerungsreaktion anfallende Rohascorbinsäure hat eine Reinheit größer 98 % und kann direkt einem der üblichen Reinigungsverfahren entweder als Feststoff oder in Lösung zugeleitet werden. Die Ausbeute nach dieser Verfahrensvariante liegt bei 90 bis 95 %.

Anhand der folgenden Beispiele soll das erfindungsgemäße Verfahren näher erläutert werden.

Die Ausbeuten und Reinheiten der beschriebenen Versuche beziehen sich auf isoliertes, getrocknetes Vitamin C. Die Ausbeuteangaben basieren auf Mol %. Die Reinheit der 2-Keto-L-gulonsäure und der Diaceton-2-keto-L-gulonsäure wurde durch HPLC kalibriert gegen eine Referenzprobe gemessen. Der Gehalt an Vitamin C wurde im Rohprodukt mit der üblichen iodometrischen Titrationsmethode ermittelt.

### Beispiel 1

In 1,5 l (17 mol) n-Butanol wurden 742 g (3,75 mol) wasserfreie 2-Keto-L-gulonsäure suspendiert und nach Zusatz von 8 g konzentrierter Schwefelsäure auf 200 mbar evakuiert. Nach Erhitzen auf 85°C wurden nach 2 Stunden 1,1 l wasserhaltiges n-Butanol abdestilliert. Die viskose, goldgelb gefärbte Schmelze wurde mit 906 ml (8,9 mol) Perchlorethylen versetzt und nach Zusatz von 84 ml konz. Salzsäure 17 Stunden bei 72 bis 75°C umgelagert. Die ausgefallene L-Ascorbinsäure wurde abgesaugt, mit n-Butanol gewaschen und im Vakuum getrocknet. Man erhielt 588 g (88 %) eines hellgrauen Rohproduktes mit einer Reinheit von 98,5 %. Das Rohprodukt löste sich in Wasser. Nach dem Klarfiltrieren erhielt man eine farblose Lösung, aus der L-Ascorbinsäure in literaturbekannter Weise isoliert werden konnte. Der Rückstand (0,9 %) bestand überwiegend aus Kohlenstoff.

### Beispiel 2

Gemäß Beispiel 1 wurde anstelle von wasserfreier 2-Keto-L-gulonsäure 2-Keto-L-gulonsäure Monohydrat eingesetzt. Die Veresterungszeit erhöhte sich nur unwesentlich. Die Menge des abdestillierten Wassers erhöhte sich im Destillat entsprechend. Die Ausbeute an L-Ascorbinsäure (Rohprodukt) betrug 87,5 % (Reinheit 98,9 %).

### Beispiel 3

Gemäß Beispiel 1 wurde anstelle von wasserfreier 2-Keto-L-gulonsäure eine 40 gew.-%ige wäßrige 2-Keto-L-gulonsäurelösung (bez. auf wasserfreie Säure) eingesetzt. Die Veresterungszeit erhöhte sich um 45 min. Die Menge des abdestillierten Wassers erhöhte sich ebenfalls uni die zusätzlich eingesetzte Menge. Die Ausbeute an L-Ascorbinsäure (Rohprodukt) betrug 87,3 % (Reinheit 98,6 %).

### Beispiel 4

In Analogie zu Beispiel 1 wurde verestert und umgelagert. Der Unterschied bestand aber darin, daß das goldgelbe Veresterungsgemisch in Perchlorethylen/Salzsäure eingerührt wurde. Die Ausbeute an getrocknetem Rohprodukt betrug 89 % (Reinheit 99,5 %).

### Beispiel 5

Gemäß Beispiel 1 wurde verestert und umgelagert. Die ausgefallene L-Ascorbinsäure wurde mit insgesamt 450 ml Wasser zweimal extrahiert und nach dem Klarfiltrieren aufgereinigt. In der wäßrigen Lösung befanden sich nach schonendem Eindampfen 87 % L-Ascorbinsäure mit einer Reinheit von 99 %.

### Beispiel 6

In einem Lösungsmittelgemisch, bestehend aus 72 ml (0,7 mol) Perchlorethylen und 1 l (11 mol) n-Butanol wurden 594 g (3 mol) wasserfreie 2-Keto-L-gulonsäure suspendiert. Nach Zusatz von 7 g (69 mmol) konz. Schwefelsäure wurde auf 300 mbar evakuiert und auf eine Innentemperatur von 85°C erhitzt. Das Destillat wurde abgetrennt. Nach 2 Stunden wurden 24 ml Wasser ausgekreist. Das zähe Reaktionsgemisch wurde nach Zusatz von 725 ml (7 mol) frischem Perchlorethylen rasch mit 79 g (0,8 mol) konz. Salzsäure versetzt und 17 Stunden bei 75°C nachgerührt. Die ausgefallene L-Ascorbinsäure wurde abgesaugt, mit n-Butanol nachgewaschen und im Vakuum getrocknet. Ausbeute: 460 g (86 %) graues Kristallisat mit einer Vitamin C Reinheit von 99 %. Durch literaturbekannte Verfahren, z.B. Umkristallisation kann Vitamin C hoher Reinheit gewonnen werden.

### Beispiel 7

In 667 g (9 mol) n-Butanol wurden 584 g (2 mol) Diaceton-2-keto-L-gulonsäure Monohydrat suspendiert und nach Zusatz von 5 g konzentrierter Schwefelsäure auf 200 mbar evakuiert. Nach Erhitzen auf 85°C wurden nach 2 Stunden 580 g aceton- und wasserhaltiges n-Butanol abdestilliert. Die viskose, goldgelb gefärbte Schmelze wurde mit 906 ml (8,9 mol) Perchlorethylen versetzt und nach Zusatz von 84 ml konz. Salzsäure 17 Stunden bei 72 bis 75°C umgelagert. Die ausgefallene L-Ascorbinsäure wurde abgesaugt, mit n-Butanol gewaschen und im Vakuum getrocknet. Man erhielt 580 g (87 %) eines hellgrauen Rohproduktes mit einer Reinheit von 98,6 %.

### Beispiel 8

Gemäß Beispiel 1 wurde die Veresterungsreaktion kontinuierlich betrieben. Die viskose, goldgelb gefärbte Schmelze wurde bei 72°C in einem kontinuierlichen Strom von 12,4 kg/Std. zusammen mit 0,1 mol 37%iger Salzsäure/Std. in einen auf 107°C erhitzten Rohreaktor gepumpt. Es stellte sich im stationären Betrieb ein Druck von 7,2 bar ein. Nach einer Verweilzet von 4 min. wurde das Reaktionsgemisch in einen mit 20-50 mbar betriebenen Dünnfilmverdampfer bei einer Temperatur von 32°C entspannt, aus dem im Sumpf eine wäßrige, fast farblose Lösung von L-Ascorbinsäure isoliert wurde. Diese wurde nach weiterer Abkühlung direkt der Reinigung und Kristallisation zugeführt. Eine Gehaltsbestimmung einer Probe des Reaktionsaustrages ergab eine Ausbeute an L-Ascorbinsäure von 87,5 % mit einer Reinheit von 98,3 %. Das im Dünnfilmverdampfer über Kopf abgezogene Destillat wurde nach Kondensation redestilliert und der zurückgewonnene Alkohol erneut zur Veresterung verwendet.

## Patentansprüche

1. Verfahren zur Herstellung von L-Ascorbinsäure, dadurch gekennzeichnet, daß man eine Schmelze von 2-Keto-L-gulonsäure-C₃-C₁₀-alkylester unter sauren Bedingungen lactonisiert.

2. Verfahren nach Anspruch 1, dadurch gekennzeichnet, daß man die Lactonisierung in einem inerten, mit Wasser nicht mischbaren Lösungsmittel in Gegenwart einer Mineralsäure durchführt.

3. Verfahren nach einem der Ansprüche 1 oder 2, dadurch gekennzeichnet, daß man die Lactonisierung in einem halogenierten Kohlenwasserstoff in Gegenwart von wäßriger Salzsäure durchführt.

4. Verfahren nach Anspruch 3, dadurch gekennzeichnet, daß man als inertes Lösungsmittel halogenierte Kohlenwasserstoffe, ausgewählt aus der Gruppe, bestehend aus Dichlormethan, Chloroform und Perchlorethylen verwendet.

5. Verfahren nach einem der Ansprüche 1 bis 4, dadurch gekennzeichnet, daß man
a) 2-Keto-L-gulonsäure oder 2,3:4,6-Di-O-isopropyliden-2-keto-L-gulonsäure in Gegenwart eines sauren Katalysators mit einem C₃-C₁₀-Alkohol verestert,
b) den überschüssigen C₃-C₁₀-Alkohol zusammen mit dem gebildeten Reaktionswasser abdestilliert und
c) anschließend den gebildeten 2-Keto-L-gulonsäure-C₃-C₁₀-alkylester in Form einer wasserfreien Schmelze unter sauren Bedingungen lactonisiert.

6. Verfahren nach Anspruch 5, dadurch gekennzeichnet, daß man die Veresterung im Verfahrensschritt a) mit einem Alkohol, ausgewählt aus der Gruppe, bestehend aus n-Propanol, Isopropanol, 1-Butanol, 2-Butanol, 2-Methyl-1-propanol, 2-Methyl-2-propanol, 1-Pentanol, 1-Hexanol und 1-Octanol durchführt.

7. Verfahren nach einem der Ansprüche 5 oder 6, dadurch gekennzeichnet, daß man die Veresterung im Verfahrensschritt a) in Gegenwart einer Mineralsäure oder eines sauren Ionenaustauschers durchführt.

8. Verfahren nach einem der Ansprüche 5 bis 7, dadurch gekennzeichnet, daß man die Veresterung im Verfahrensschritt a) mit n-Butanol in Gegenwart von Schwefelsäure durchführt.

9. Verfahren nach einem der Ansprüche 5 bis 8, dadurch gekennzeichnet, daß man die Veresterung im Verfahrensschritt a) in einem inerten, mit Wasser nicht mischbaren Lösungsmittel durchführt.

10. Verfahren nach einem der Ansprüche 5 bis 9, dadurch gekennzeichnet, daß man als Ausgangsmaterial 2-Keto-L-gulonsäure verwendet.

11. Verfahren nach einem der Ansprüche 5 bis 10, dadurch gekennzeichnet, daß die Reaktionstemperaturen in den Verfahrensschritten a) bis c) im Bereich von -10 bis 160°C liegen.

12. Verfahren nach einem der Ansprüche 5 bis 11, dadurch gekennzeichnet, daß die Veresterung und Lactonisierung in den Verfahrensschritten a) bis c) bei Drücken im Bereich von 0,1 bis 10 bar erfolgen.

13. Verfahren nach einem der Ansprüche 5 bis 12, dadurch gekennzeichnet, daß der in den Verfahrensschritten a) und b) gebildete Ester ohne Isolierung und Aufreinigung direkt in die Lactonisierungsstufe eingesetzt wird.
